(19) **Europäisches Patentamt / European Patent Office / Office européen des brevets**

(11) **EP 3 604 501 A1**

(12) **EUROPEAN PATENT APPLICATION**
published in accordance with Art. 153(4) EPC

(43) Date of publication:
**05.02.2020 Bulletin 2020/06**

(51) Int Cl.:
**C12N 5/0797** *(2010.01)*

(21) Application number: **18771505.7**

(22) Date of filing: **22.03.2018**

(86) International application number:
**PCT/JP2018/011492**

(87) International publication number:
**WO 2018/174186 (27.09.2018 Gazette 2018/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.03.2017 JP 2017057635**

(71) Applicants:
• **UBE Industries, Ltd.**
**Ube-shi, Yamaguchi 755-8633 (JP)**
• **National University Corporation Chiba University**
**Chiba-shi, Chiba 267-8522 (JP)**

(72) Inventors:
• **KASUYA, Yoshitoshi**
**Chiba-shi**
**Chiba 260-8670 (JP)**
• **YOSHIOKA, Kento**
**Chiba-shi**
**Chiba 260-8670 (JP)**
• **HAGIHARA, Masahiko**
**Ube-shi**
**Yamaguchi 755-8633 (JP)**

(74) Representative: **Plougmann Vingtoft a/s**
**Strandvejen 70**
**2900 Hellerup (DK)**

(54) **METHOD FOR INHIBITING DIFFERENTIATION OF NEURAL STEM CELL, METHOD FOR PREPARING NEURAL STEM CELL, AND METHOD FOR DIFFERENTIATING AND INDUCING NEURAL STEM CELL**

(57) The present invention relates to: a method for inhibiting the differentiation of a neural stem cell by culturing the neural stem cell on a polymer porous membrane; a method for preparing a neural stem cell; and a method for differentiating and inducing a neural stem cell.

**Description**

FIELD

The present invention relates to a method for inhibiting differentiation of neural stem cells, a method of preparing neural stem cells, and a method for inducing differentiation of neural stem cells.

BACKGROUND

Regarding culturing of neural stem cells

Neural stem cells have the ability to self-replicate and to differentiate into neurons and glial cells and thus have been receiving attention in the field of nerve regenerative medicine. However, culturing neural stem cells over a long period while retaining the ability thereof to differentiate is known to be difficult. For example, it has been reported that continued subculturing of wild type neural cells derived from an adult hippocampus significantly decreases the ability to self-replicate and the pluripotency thereof (NPL 1).
It has been reported that undifferentiated neural stem cells can be prepared that have not formed aggregates (have not formed spheroids) by using a honeycomb-like porous body (PTL 1). The honeycomb-like porous body used in Patent Literature 1 refers to a thin membrane structure in which micropores (encompasses recesses) are formed in the direction perpendicular to the membrane with a honeycomb-like shape in the plane of the membrane.

Porous polyimide film

Porous polyimide films have been utilized in the prior art for filters and low permittivity films, and especially for battery-related purposes, such as fuel cell electrolyte membrane and the like. PTLs 2 to 4 describe porous polyimide films with numerous macrovoids, having excellent permeability to objects such as gases, high porosity, excellent smoothness on both surfaces, relatively high strength and, despite high porosity, excellent resistance against compression stress in the film thickness direction. All of these are porous polyimide films formed via amic acid.
The cell culture method which includes applying cells to a porous polyimide film and culturing them is reported (PTL 5).

[CITATION LIST]

[PATENT LITERATURE]

[PTL 1] Japanese Unexamined Patent Publication (Kokai) No. 2008-054621
[PTL 2] WO2010/038873
[PTL 3] Japanese Unexamined Patent Publication (Kokai) No. 2011-219585
[PTL 4] Japanese Unexamined Patent Publication (Kokai) No. 2011-219586
[PTL 5] WO2015/012415

[NON-PATENT LITERATURE]

[NPL 1] Seaberg, M. et al., The Journal of Neuroscience, March 1, 2002, 22(5):1784-1793

SUMMARY

[TECHNICAL PROBLEM]

The present invention addresses the problem of providing a method that can supply a large amount of neural stem cells while inhibiting the differentiation thereof using a completely different means from the prior art.

[SOLUTION TO PROBLEM]

In order to solve the aforementioned problem, the present inventors, through extensive research, cultured neural stem cells on a three-layer structure porous polymer film having two surface layers having a plurality of pores

and a macrovoid layer sandwiched between the two surface layers, discovering the surprising fact that neural stem cell differentiation was inhibited, and thereby arriving at the present invention. The porous polymer film has a three-dimensional structure that is completely different from the honeycomb-like porous body disclosed in Patent Literature 1.

Specifically, the present invention has the following aspects.

[1] A method for inhibiting differentiation of neural stem cells comprising the steps:

  (1) applying neural stem cells to a porous polymer film; and
  (2) culturing and proliferating the neural stem cells,

wherein the porous polymer films are a three-layer structure porous polymer film having a surface layer A and a surface layer B, the surface layers having a plurality of pores, and a macrovoid layer sandwiched between the surface layers A and B, wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by such a partition wall and the surface layers A and B; and wherein the pores in the surface layers A and B communicate with the macrovoid.

[2] The method according to [1], wherein the neural stem cells are wild-type cells.

[3] The method according to [1] or [2], wherein the step (2) is implemented for at least 70 days.

[4] The method according to any one of [1] to [3], wherein the step (2) is implemented until the neural stem cells have proliferated to at least $1.0 \times 10^5$ per square centimeter of the porous polymer film.

[5] The method according to any one of [1] to [4], using two or more porous polymer films layered either above and below or left and right in the cell culture medium.

[6] The method according to any one of [1] to [5], wherein the porous polymer film is:

  i) folded;
  ii) wound into a roll;
  iii) connected as sheets or fragments by a filamentous structure, or
  iv) bound into a rope,

and suspended or anchored in a cell culture medium in a cell culturing vessel.

[7] The method according to any one of [1] to [6], wherein in the step (2), the total volume of the cell culture medium contained in the cell culturing vessel is 10,000 times or less than the total volume of the porous polyimide film including a cell survival zone.

[8] The method according to any one of [1] to [7], wherein the average pore diameter of the surface layer A is 5 $\mu$m to 50 $\mu$m.

[9] The method according to any one of [1] to [8], wherein the average pore diameter of the surface layer B is 20 to 100 $\mu$m.

[10] The method according to any one of [1] to [9], wherein the thickness of the porous polymer film is 5 to 500 $\mu$m.

[11] The method according to any one of [1] to [10], wherein the porous polymer film is a porous polyimide film.

[12] The method according to [11], wherein the porous polyimide film is a porous polyimide film comprising a polyimide derived from tetracarboxylic acid dianhydride and diamine.

[13] The method according to [11] or [12], wherein the porous polyimide film is a colored porous polyimide film that is obtained by molding a polyamic acid solution composition comprising a polyamic acid solution derived from tetracarboxylic dianhydride and diamine, and a coloring precursor, and subsequently heat-treating the resultant composition at 250°C or higher.

[14] The method according to any one of [1] to [10], wherein the porous polymer film is a porous polyethersulfone film.

[15] A method for preparing neural stem cells comprising the steps:

  (1) applying neural stem cells to a porous polymer film; and
  (2) culturing and proliferating the neural stem cells,

wherein the porous polymer films are a three-layer structure porous polymer film having a surface layer A and a surface layer B, the surface layers having a plurality of pores, and a macrovoid layer sandwiched between the surface layers A and B, wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by such a partition wall and the surface layers A and B; wherein the pores in the surface layers A and B communicate with the macrovoid;

and wherein the differentiation of neural stem cells is inhibited in the step (2).

[16] The method according to [15], further comprising the step of harvesting neural stem cells obtained in the step (2).

[17] A method for inducing differentiation of neural stem cells comprising the steps:

(1) applying neural stem cells to a porous polymer film;
(2) culturing and proliferating the neural stem cells; and
(3) culturing the neural stem cells obtained in the step (2) under differentiation-inducing conditions

wherein the porous polymer films are a three-layer structure porous polymer film having a surface layer A and a surface layer B, the surface layers having a plurality of pores, and a macrovoid layer sandwiched between the surface layers A and B, wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by such a partition wall and the surface layers A and B; and wherein the pores in the surface layers A and B communicate with the macrovoid; and wherein the differentiation of neural stem cells is inhibited in the step (2).

[ADVANTAGEOUS EFFECTS OF INVENTION]

According to the present invention, the differentiation of neural stem cells can be inhibited and a large amount of the cells can be provided.

BRIEF DESCRIPTION OF DRAWINGS

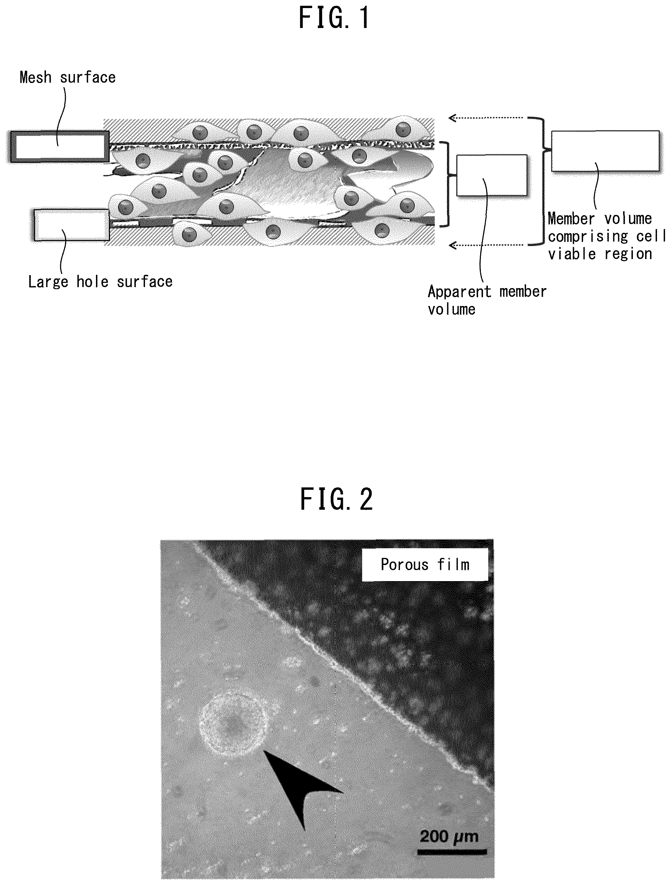

FIG. 1 represents a diagram illustrating a cell culture model using a porous polyimide film.

FIG. 2 represents a diagram illustrating an optical micrograph of a neural stem cell spheroid 10 days after the start of culturing on a porous polyimide film.

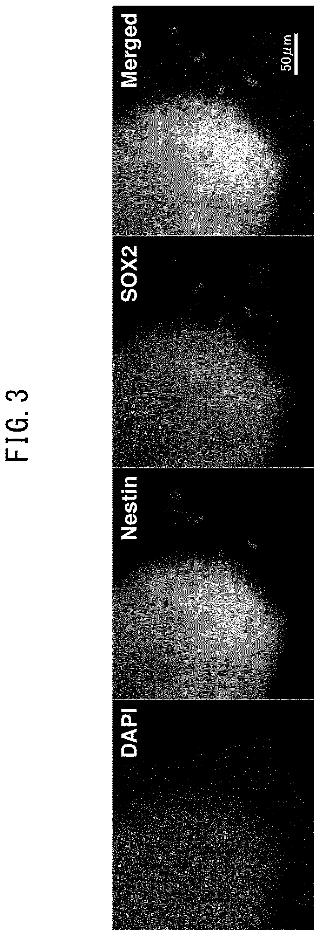

FIG. 3 represents a diagram illustrating photomicrographs of immunostained neural stem cell spheroids released from a porous polyimide film into a culture medium 10 days after the start of culturing. 4',6-diamidino-2-phenylindole (DAPI) (nuclear staining marker): blue, Nestin (marker for neural stem cells): green, SOX2 (marker for neural stem cells): red.

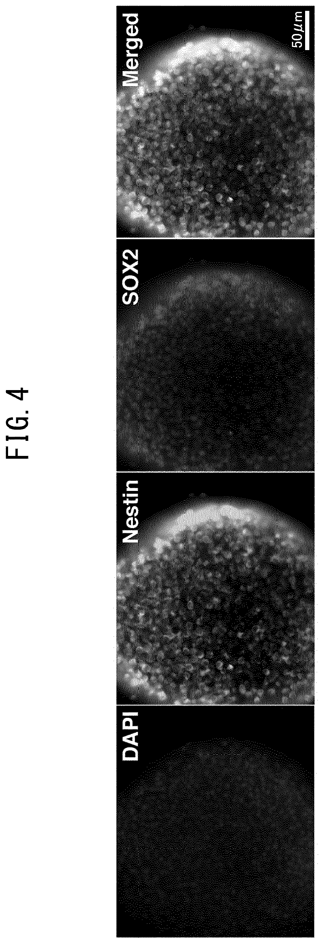

FIG. 4 represents a diagram illustrating photomicrographs of immunostained neural stem cell spheroids released from a porous polyimide film into a culture medium 70 days after the start of culturing. DAPI: blue, Nestin: green, SOX2: red.

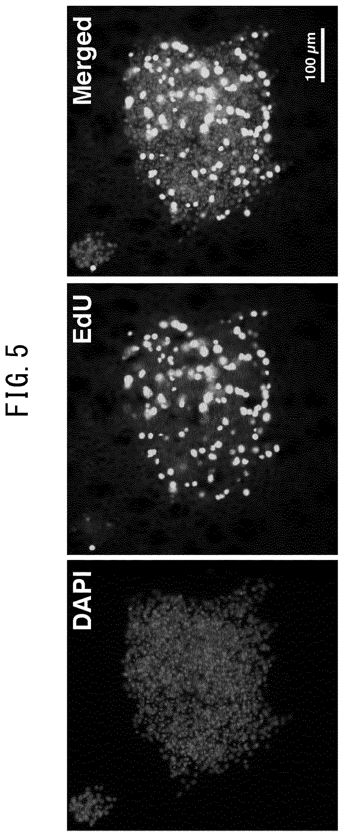

FIG. 5 represents a diagram illustrating photomicrographs (middle and right), in which 30 days after the start of culturing, EdU (5-ethynyl-2'-deoxyuridine) was incorporated as an indicator of DNA synthesis into neural stem cell aggregates cultured inside and on a porous polyimide film, and the EdU was then fluorescently labelled. Photomicrographs (left and right) in which cell nuclei were immunostained with DAPI are also shown.

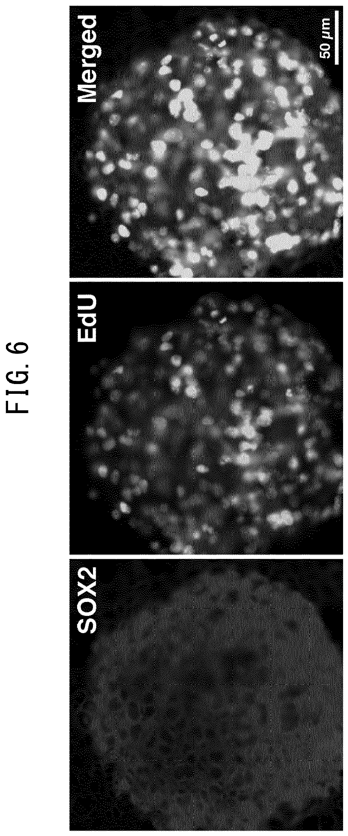

FIG. 6 represents a diagram illustrating photomicrographs (middle and right), in which 30 days after the start of culturing, EdU was incorporated as an indicator of DNA synthesis into neural stem cell aggregates cultured within and on a porous polyimide film, and the EdU was then fluorescently labelled. Photomicrographs (left and right) in which SOX2 was immunostained are also shown.

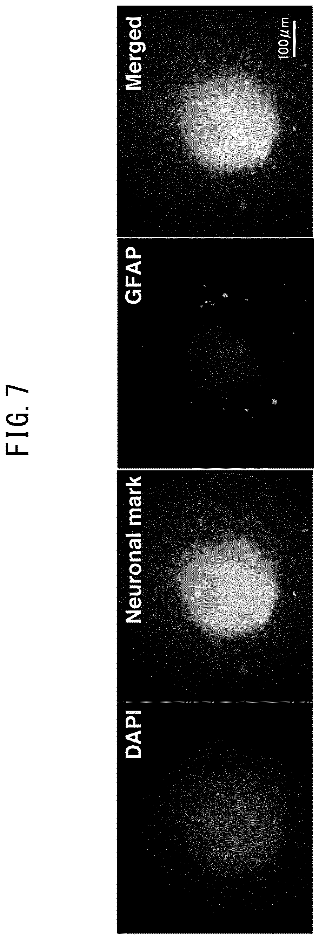

FIG. 7 represents a diagram illustrating photomicrographs, in which a differentiation inducing agent platelet-derived growth factor (PDGF) was added to neural stem cell spheroids released from a porous polyimide film on the tenth day of culturing, the culturing was carried out for five days, and then immunostaining was performed. DAPI: blue, Milli-Mark Pan Neuronal Marker™ (Neuronal mark) (marker for mature neurons): green, glial fibrillary acidic protein (GFAP) (molecular marker for astrocytes): red.

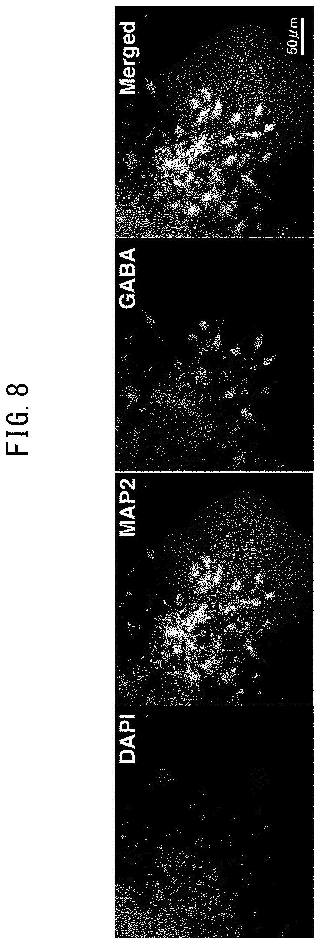

FIG. 8 represents a diagram illustrating photomicrographs, in which a differentiation inducing agent forskolin was added to neural stem cell spheroids released from a porous polyimide film on the tenth day of culturing, the culturing was subsequently carried out for five days, and then immunostaining was performed. DAPI: blue, microtubule-associated protein 2 (MAP2) (marker for neuronal dendrites): green, $\gamma$-aminobutyric acid (GABA) (molecular marker for GABAergic neurons): red.

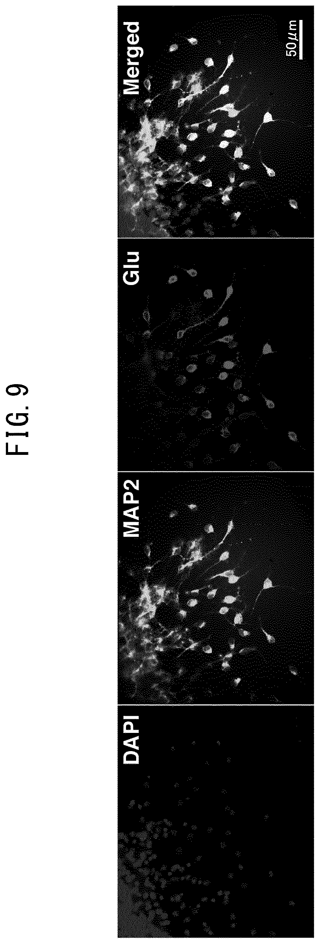

FIG. 9 represents a diagram illustrating photomicrographs, in which a differentiation inducing agent forskolin was added to neural stem cell spheroids released from a porous polyimide film on the tenth day of culturing, the culturing was subsequently carried out for five days, and then immunostaining was performed. DAPI: blue, MAP2: green, Glu (glutamic acid) (molecular marker of glutaminergic neurons): red.

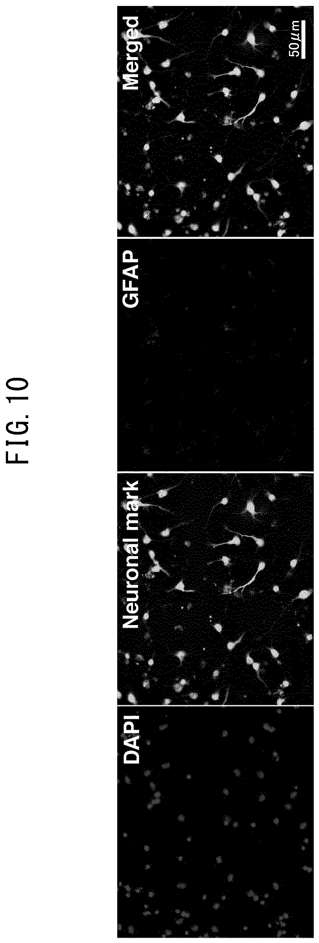

FIG. 10 represents a diagram illustrating photomicrographs, in which a differentiation inducing agent PDGF was added to neural stem cell spheroids released from a porous polyimide film on the 70th day of culturing, the culturing was subsequently carried out for five days, and then immunostaining was performed. DAPI: blue, Neuronal mark: green, GFAP: red.

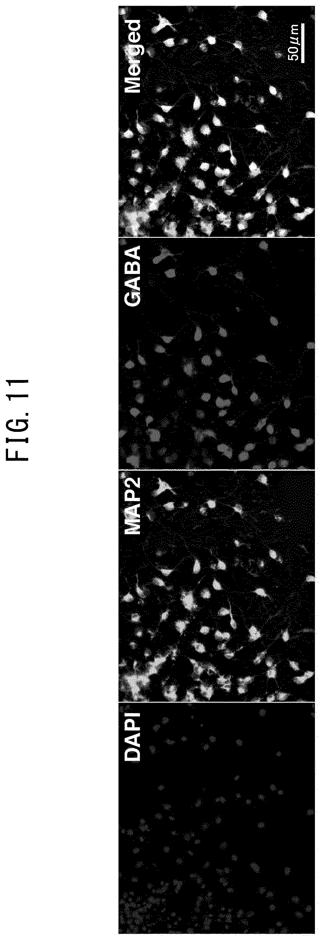

FIG. 11 represents a diagram illustrating photomicrographs, in which a differentiation inducing agent forskolin was added to neural stem cell spheroids released from a porous polyimide film on the 70th day of culturing, the culturing was subsequently carried out for five days and then immunostaining was performed. DAPI: blue, MAP2: green, GABA: red.

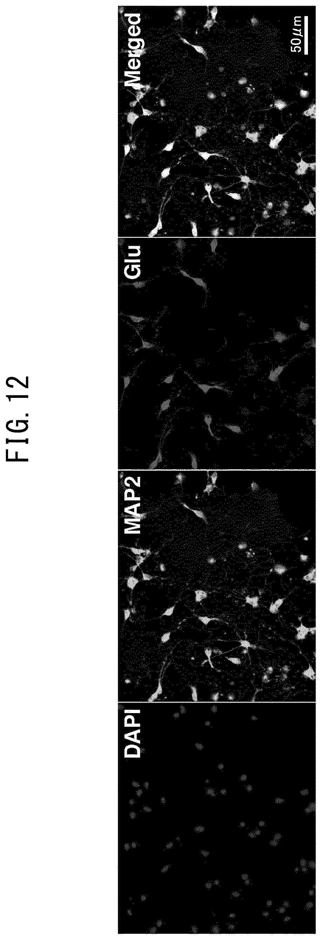

FIG. 12 represents a diagram illustrating photomicrographs, in which a differentiation inducing agent forskolin was added to neural stem cell spheroids released from a porous polyimide film on the 70th day of culturing, the culturing was subsequently carried out for five days and then immunostaining was performed. DAPI: blue, MAP2: green, Glu: red.

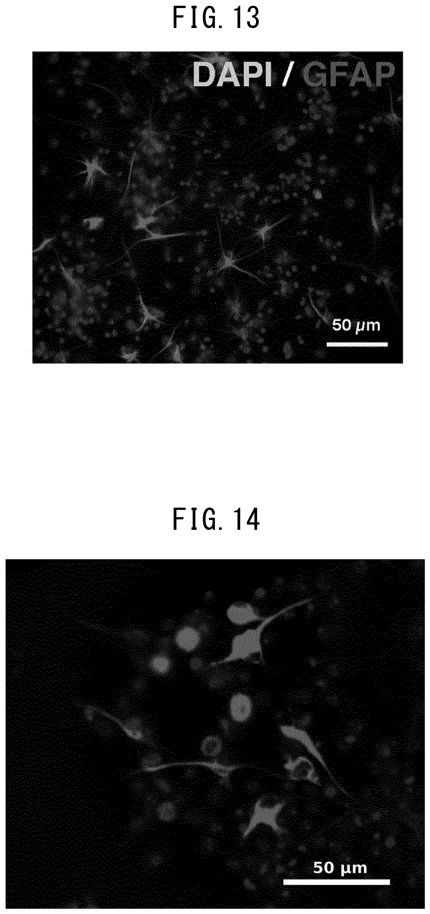

FIG. 13 represents a diagram illustrating a photomicrograph, in which a differentiation inducing agent bone morphogenetic protein 2 (BMP2) predominantly for astrocytes was added to neural stem cell spheroids released from a porous polyimide film on the 70th day of culturing, the culturing was subsequently carried out for five days and then immunostaining was performed. DAPI: blue, GFAP: red.

FIG. 14 represents a diagram illustrating a photomicrograph, in which BMP2 (100 ng/mL) was added to neural stem cell spheroids released from a porous polyimide film on the 225th day of culturing, culturing was subsequently carried out for five days and then immunostaining was performed. DAPI: blue, GFAP: red.

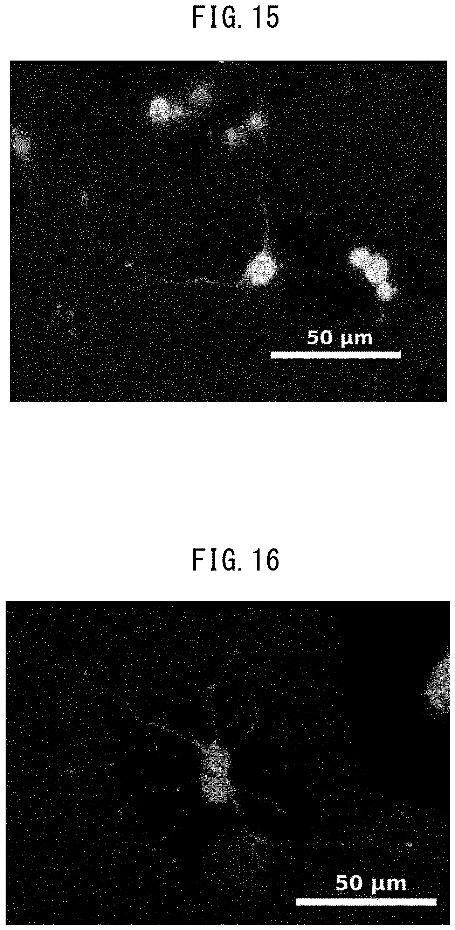

FIG. 15 represents a diagram illustrating a photomicrograph, in which a differentiation inducing agent PDGF (10 ng/mL) was added to neural stem cell spheroids released from a porous polyimide film on the 225th day of culturing, the culturing was subsequently carried out for five days and then immunostaining was performed. GFAP: red, MAP2: green, DAPI: blue.

FIG. 16 represents a diagram illustrating a photomicrograph, in which a differentiation inducing agent PDGF (10 ng/mL) was added to neural stem cell spheroids released from a porous polyimide film on the 225th day of culturing, the culturing was subsequently carried out for five days and then immunostaining was performed. NG2 (molecular marker for juvenile oligodendrocytes): red, O4 (molecular marker for mature oligodendrocytes): green, DAPI: blue.

DESCRIPTION OF EMBODIMENTS

One aspect of the present invention relates to a method for inhibiting differentiation of neural stem cells comprising the steps:

(1) applying neural stem cells to a porous polymer film; and
(2) culturing and proliferating the neural stem cells,

wherein the porous polymer films are a three-layer structure porous polymer film having a surface layer A and a surface layer B, the surface layers having a plurality of pores, and a macrovoid layer sandwiched between the surface layers A and B, wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by such a partition wall and the surface layers A and B; and wherein the pores in the surface layers A and B communicate with the macrovoid. Hereinafter, this is also referred to as the "method for inhibiting differentiation according to the present application".

Another embodiment of the present invention relates to a method of preparing neural stem cells comprising the steps:

(1) applying neural stem cells to a porous polymer film; and
(2) culturing and proliferating neural stem cells;
wherein the porous polymer films are a three-layer structure porous polymer film having a surface layer A and a surface layer B, the surface layers having a plurality of pores, and a macrovoid layer sandwiched between the surface layers A and B, wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by such a partition wall and the surface layers A and B; and wherein the pores in the surface layers A and B communicate with the macrovoid, and the differentiation of the neural stem cells being inhibited in step (2). Hereinafter, this is also referred to as the "preparation method of the present invention".

Another embodiment of the present invention relates to a method of inducing the differentiation of neural stem cells comprising the steps:

(1) applying neural stem cells to a porous polymer film;
(2) culturing and proliferating the neural stem cells: and
(3) culturing the neural stem cells obtained in step (2) under differentiation-inducing conditions;

wherein the porous polymer films are a three-layer structure porous polymer film having a surface layer A and a surface layer B, the surface layers having a plurality of pores, and a macrovoid layer sandwiched between the surface layers A and B, wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by such a partition wall and the surface layers A and B; and wherein the pores in the surface layers A and B communicate with the macrovoid, and the differentiation of the neural stem cells being inhibited in step (2). Hereinafter this is also referred to as the "method for inducing differentiation according to the present invention".

Hereinafter, the "method of inhibiting differentiation according to the present invention", the "preparation method of the present invention" and the "method of inducing differentiation according to the present invention" are also referred to as the "methods of the present invention".

1. Regarding neural stem cells used in the methods of the present invention

The "neural stem cells" disclosed herein are cells having the ability to self-replicate and the ability to differentiate into neurons and glial cells.

The type of neural stem cells that can be used in the present invention is not particularly limited. However, mammalian neural stem cells are preferable, more preferable are neural stem cells from primates (humans, monkeys, etc.), rodents (mice, rats, guinea pigs, etc.), cats, dogs, rabbits, sheep, pigs, cattle, horses, donkeys, goats, and ferrets, particularly preferable are human neural stem cells.

The method of obtaining the neural stem cells used in the methods of the present invention are not particularly limited. For example, the neural stem cells may be isolated from tissue of the central nervous system of an animal. It is preferable that the tissue of the central nervous system be tissue of the hippocampus, lateral ventricle and the surrounding area. Furthermore, the stage of growth and development of the animal is not particularly limited and may be a fetus, immature, or an adult, etc. Alternatively, the neural stem cells used in the methods of the present invention may be obtained by inducing the differentiation of pluripotent stem cells such as embryonic stem cells (ES cell), embryonal carcinoma cells (EC cells), embryonic germ cells (EG cells), nuclear transfer ES cells, somatic cell-derived ES cells (ntES cells), induced pluripotent stem cells (iPS cells), and Muse cells (Multi-lineage differentiating stress enduring cell).

The neural stem cells used in the methods of the present invention may be wild type cells or mutant cells (for example, p38α heterozygous mouse-derived cells (refer to Yoshioka, K. et al., FEBS Open Bio 5(2015)437-444)).

2. Regarding the step of applying the neural stem cells used in the present invention onto a porous polymer film

Although in no way limited, the application of the neural stem cells onto the porous polymer film in the methods of the present invention include, for example, the following embodiments.

(A) An embodiment comprising a step of inoculating neural stem cells onto the surface of the porous polymer film.
(B) An embodiment comprising the steps of putting a neural stem cell suspension onto a dried surface of a porous polymer film, making the neural stem cell suspension be absorbed into the film by leaving the porous polymer film to stand, moving the porous polymer film to promote the drainage of liquid, or stimulating a part of the surface of the film, then retaining the neural stem cells of the neural stem cell suspension in the film and draining the moisture.
(C) An embodiment comprising a step of wetting one side or both sides of the porous polymer film with cell culture solution or a sterile solution, loading a neural stem cell suspension onto the wet polymer film, then retaining the neural stem cells in the neural stem cell suspension in the film and draining the moisture.

Embodiment (A) includes the direct inoculation of cells or cell aggregates onto the surface of a porous polymer film or the immersion of the porous polymer film into a neural stem cell suspension such that the cell culture solution permeates the film through the surface thereof.

The neural stem cells inoculated on the surface of the porous polymer film adhere to the porous polymer film and enter the pores. Preferably, the neural stem cells spontaneously adhere to the porous polymer film without the addition of an external physical or chemical force. The neural stem cells inoculated on the surface of the porous polymer film can stably grow and proliferate on the surface and/or inside the film. The neural stem cells can assume various forms depending on the position of the film on which the cells are grown and made to proliferate on.

In embodiment (B), a neural stem cell suspension is put onto the dried surface of a porous polymer film. The neural stem cell suspension is made to permeate the film by leaving the porous polymer film to stand, moving the porous polymer film to promote the drainage of liquid therefrom, or stimulating a part of the surface such that the neural stem cell suspension is absorbed into the film. Although not bound by theory, this is thought to depend on properties derived from, for example, the shape of each surface of the porous polymer film. According to the present embodiment, parts of the film loaded with neural stem cell suspension absorb the neural stem cell and are inoculated therewith.

Alternatively, as in embodiment (C), a part or the whole of one or both sides of the porous polymer film is wet with cell culture solution or a sterilized solution and thereafter the neural stem cell suspension may be loaded into the wet porous polymer film. In such cases, the rate of permeation of the neural stem cell suspension is greatly increased.

For example, mainly for the purpose of preventing dispersion in the film, a method of wetting a tiny part of the film can be used (hereinafter, referred to as "single point wetting method"). The single point wetting method is essentially nearly the same as the dry method (embodiment (B)) in which the film is not wetted. However, it is considered that the cell solution quickly permeates the film in the small wetted part. Furthermore, a method for loading a neural stem cell suspension into a porous polymer film, the whole of one or both sides of which has been sufficiently wetted (hereinafter referred to as "wet film") may also be used (hereinafter referred to as "wet film method"). In such cases, the rate of permeation of the neural stem cell suspension in the whole porous polymer film is greatly increased.

In embodiments (B) and (C), the neural stem cells in the neural stem cell suspension are retained in the film and the moisture is drained. The concentration of the neural stem cells in the neural stem cell suspension is thereby increased, components other than the neural stem cells that are not required are drained with the moisture, and other such treatment can be carried out.

Embodiment (A) may be referred to as "natural inoculation" and embodiments (B) and (C) may be referred to as "absorption inoculation".

Although in no ways limited, it is preferable that living cells are selectively retained in the porous polymer film. Therefore, in a preferable embodiment of the method of the present invention, living cells are retained in the porous polymer film while dead cells are preferentially drained together with the moisture.

The sterilized solution used in embodiment (C) is not particularly limited but may be a sterilized buffer solution or sterilized water. The buffer solution is, for example, (+) and (-) Dulbecco's PBS or (+) and (-) Hank's Balanced Salt Solution. Examples of the buffer solution are listed in Table 1 below.

[Table 1]

| Component | Concentration (mmol/L) | Concentration (g/L) |
|---|---|---|
| NaCl | 137 | 8.00 |
| KCL | 2.7 | 0.20 |
| $Na_2HPO_4$ | 10 | 1.44 |
| $KH_2PO_4$ | 1.76 | 0.24 |
| pH (-) | 7.4 | 7.4 |

Furthermore, in the method of the present invention, the application of neural stem cell on the porous polymer film includes an embodiment (entanglement) in which neural stem cells are made to attach to the film by a suspension of neural stem cells being made to exist together in suspension with the porous polymer film. For example, in the methods of the present invention, in order to apply the neural stem cells to the porous polymer film, a cell culture medium, neural stem cells and one or more of the porous polymer film may be loaded into a cell culture vessel. In the case of the cell culture medium being a liquid, the porous polymer film is suspended in the cell culture medium. The neural stem cells can adhere to the porous polymer film due to the properties of the porous polymer film. Thus, culturing is possible on the porous polymer film while suspended in cell culture medium. Preferably, the neural stem cells spontaneously adhere to the porous polymer film. "Spontaneously adhere to" is defined as the neural stem cells being retained on the surface or within the porous polymer film without the addition of an external physical or chemical force.

The aforementioned application of the neural stem cells onto the porous polymer film can involve the combination of two or more methods. For example, of embodiments (A) to (C), a combination of two or more methods can be used to apply the neural stem cells to the porous polymer film.

3. Regarding the step of culturing and proliferating neural stem cells used in the present invention

Culturing of neural stem cells used in the present invention may be carried out by adherent culture in which cells adhere to the porous polymer film or may be carried out by suspension culture in which cells are suspended in a culture medium. Suspension culture is preferable from the viewpoint of allowing large-scale culture.

In the method of the invention, when the porous polymer film is used in a state suspended in the cell culture medium, two or more fragments of the porous polymer film may be used. Since the porous polymer film is a three-dimensional, flexible thin-film, using such fragments that are suspended in the culture solution, for example, allows a porous polymer film with a large culturable surface area to be added into a fixed volume of cell culture medium. In the case of normal culturing, the container base area constitutes the area limit in which cell culture can be accomplished, but with cell culturing using the porous polymer film of the invention, all of the large surface area of the previously added porous polymer film constitutes area in which cell culturing can be accomplished. The porous polymer film allows the cell culture solution to pass through, allowing supply of nutrients, oxygen and the like even into the folded film, for example. In addition, since the porous polymer film is completely different from a conventional plate culture in that it is a cell culturing substrate having a three-dimensional and flexible structure, it allows culturing of cells with an adhering property in culturing vessels of various shapes, materials and sizes (for example, dishes, flasks, tanks or bags), regardless of the shape of the culturing vessel.

The sizes and shapes of the porous polymer film fragments are not particularly restricted. The shapes may be as desired, such as circular, elliptical, quadrilateral, triangular, polygonal or string-like.

Because the porous polymer film of the invention is flexible, it can be used with varying shapes. Instead of a flat form, the porous polymer film can also be used by working into a three-dimensional shape. For example, porous polymer films may be: i) folded, ii) wound into a roll, iii) connected as sheets or fragments by a filamentous structure, or iv) bound into a rope, for suspension or fixing in the cell culture medium in the cell culturing vessel. By forming into shapes such as i) to iv), it is possible to place a large amount of porous polymer films into a fixed volume of cell culture medium, similar to using fragments. Furthermore, since each fragment can be treated as an aggregate, it is possible to aggregate and move the cell masses together, for overall high applicability.

With the same concept as fragment aggregates, two or more porous polymer films may be used in a layered form either above and below or left and right in the cell culture medium. Layering includes a mode in which portions of the porous polymer films overlap. Layered culturing allows culturing of cells at high density in a narrow space. It is also possible to further layer a film on a film on which cells are already growing, setting it to create a multilayer of different cell types. The number of layered porous polymer films is not particularly restricted.

In the method of the present invention, it is preferable for the neural stem cells to grow and proliferate on the surface of and within the porous polymer film.

In the method of the present invention, neural stem cells can be cultured while inhibiting differentiation over long periods of at least 10 days, at least 20 days, at least 30 days, at least 50 days, at least 70 days, at least 120 days, at least 200 days, at least 225 days, and at least 300 days without performing conventional subculturing in which trypsin treatment and the like are carried out. Furthermore, according to the method of the present invention, neural stem cells can be cultured while inhibiting differentiation for periods longer than can be cultured by conventional planar culture, for example, for periods which are at least 1.5 times, at least 2 times, at least 2.5 times, at least 3 times, at least 3.5 times, at least 4 times, and at least 4.5 times that of the planar culture period. According to the present invention, neural stem cells that are active and not quiescent and that retain the original characteristics can be maintained over long periods without deformation of spheroids or the loss of the ability to differentiate, etc., that occur when culturing cells over a long period by planar culture on a petri dish or the like. Further, according to the present invention, even if the neural stem cells are cultured over a long period, cell viability and properties of the neural stem cells (e.g. the level of expression of cell surface markers and the like) remain mostly unchanged compared to the neural stem cells before culturing over the long period. Furthermore, according to the present invention, as neural stem cells proliferate within the porous polymer film three-dimensionally, the limitations on the culture region that can be seen in conventional planar culture and contact inhibition that occurs in planar environments is unlikely to occur, and thus cultures can be grown over a long period. Moreover, according to the present invention, by bringing a different porous polymer film into contact with the porous polymer film to which neural stem cells are attached, the space available for cell culture can be arbitrarily increased which allows for proliferative culturing over a long period without performing conventional subculturing in which trypsin treatment and the like are carried out and avoiding a confluent state that gives rise to contact inhibition. Furthermore, according to the present invention, a novel method of preserving cells is provided in which living neural stem cells are preserved over a long period without performing freezing or the like.

4. Regarding porous polymer film used in the present invention

The average pore diameter of pores present in the surface layer A (hereinafter may be referred to as "surface A" or "mesh surface") of the porous polymer film used in the present invention is not particularly limited. However, for example, the lower limit thereof is 0.01 $\mu$m or more, 0.1$\mu$m or more, 1 $\mu$m or more, 2 $\mu$m or more, 3 $\mu$m or more, 4 $\mu$m or more, 5 $\mu$m or more, 6 $\mu$m or more, 7 $\mu$m or more, 8 $\mu$m or more, 9 $\mu$m or more, or 10 $\mu$m or more, and the upper limit thereof is less than 200 $\mu$m, 150 $\mu$m or less, 100 $\mu$m or less, 50 $\mu$m or less, 40 $\mu$m or less, 30 $\mu$m or less, 20 $\mu$m

or less or 15 μm or less. The average pore diameter of pores present in the surface layer A is preferably 0.01 μm to 50 μm, more preferably 1 μm to 50 μm, and particularly preferably 5 μm to 50 μm.

The average pore diameter of the pore present on a surface layer B (hereinafter may be referred to as "surface B" or "large pore surface") of the porous polymer film used in the present invention is not particularly limited provided the average pore diameter is greater than for the pores present in surface layer A. However, for example, the lower limit thereof is greater than 5 μm, 20 μm or more, 30 μm or more, 40 μm or more, 50 μm or more, or 60 μm or more, and the upper limit therefor is 200 μm or less, 150 μm or less, 100 μm or less, 90 μm or less, 80 μm or less, or 70 μm or less. The average pore diameter of the pores present in surface layer B is preferably 20 μm to 100 μm.

The average pore diameter on the surface of the porous polymer film is determined by measuring pore area for 200 or more open pore portions, and calculated an average diameter according to the following Equation (1) from the average pore area assuming the pore shape as a perfect circle.

[Math. 1]

$$\text{Average Pore Diameter} = 2 \times \sqrt{(Sa / \pi)} \qquad (1)$$

(wherein Sa represents the average value for the pore areas)

The thicknesses of the surface layers A and B are not particularly limited, but is, for example, 0.01 to 50 μm, preferably 0.01 to 20 μm.

The average pore diameter of macrovoids in the planar direction of the film in the macrovoid layer in the porous polymer film is not particularly limited but is, for example, 10 to 500 μm, preferably 10 to 100 μm, and more preferably 10 to 80 μm. The thicknesses of the partition wall in the macrovoid layer are not particularly limited, but is, for example, 0.01 to 50 μm, preferably 0.01 to 20 μm. In an embodiment, at least one partition wall in the macrovoid layer has one or two or more pores connecting the neighboring macrovoids and having the average pore diameter of 0.01 to 100 μm, preferably 0.01 to 50 μm. In another embodiment, the partition wall in the macrovoid layer has no pore.

The total film thickness of the porous polymer film used for the invention is not particularly limited, but may be 5 μm or more, 10 μm or more, 20 μm or more or 25 μm or more, and 500 μm or less, 300 μm or less, 100 μm or less, 75 μm or less, or 50 μm or less. It is preferably 5 to 500 μm, and more preferably 25 to 75 μm.

The film thickness of the porous polymer film used for the invention can be measured using a contact thickness gauge.

The porosity of the porous polymer film used in the present invention is not particularly limited but is, for example, 40% or more and less than 95%.

The porosity of the porous polymer film used for the invention can be determined by measuring the film thickness and mass of the porous film cut out to a prescribed size, and performing calculation from the basis weight according to the following Equation (2).

[Math. 2]

$$\text{Porosity (\%)} = (1 - w / (S \times d \times D)) \times 100 \qquad (2)$$

(wherein S represents the area of the porous film, d represents the total film thickness, w represents the measured mass, and D represents the polymer density. The density is defined as 1.34 g/cm$^3$ when the polymer is a polyimide.)

The porous polymer film used for the present invention is preferably a porous polymer film which includes a three-layer structure porous polymer film having a surface layer A and a surface layer B, the surface layers having a plurality of pores, and a macrovoid layer sandwiched between the surface layers A and B; wherein the average pore diameter of the pore present on the surface layer A is 0.01 μm to 15 μm, and the average pore diameter of the pore present on the surface layer B is 20 μm to 100 μm; wherein the macrovoid layer has a partition wall bonded to the surface layers A and B, and a plurality of macrovoids surrounded by such a partition wall and the surface layers A and B, the thickness of the macrovoid layer, and the surface layers A and B is 0.01 to 20 μm; wherein the pores on the surface layers A and B communicate with the macrovoid, the total film thickness is 5 to 500 μm, and the porosity is 40% or more and less than 95%. In an embodiment, at least one partition wall in the macrovoid layer has one or two or more pores connecting the neighboring macrovoids with each other and having the average pore diameter of 0.01 to 100 μm, preferably 0.01 to 50 μm. In another embodiment, the partition wall does not have such pores.

The porous polymer film used for the present invention is preferably sterilized. The sterilization treatment is not particularly limited, but any sterilization treatment such as dry heat sterilization, steam sterilization, sterilization with a disinfectant such as ethanol, electromagnetic wave sterilization such as ultraviolet rays or gamma rays, and the like can be mentioned.

The porous polymer film used for the present invention is not particularly limited so long as it has the structural features described above and includes, preferably a porous polyimide film or porous polyethersulfone (PES) film.

Polyimide is a general term for polymers containing imide bonds in the repeating unit, and usually it refers to an aromatic polyimide in which aromatic compounds are directly linked by imide bonds. An aromatic polyimide has an aromatic-aromatic conjugated structure via an imide bond, and therefore has a strong rigid molecular structure, and since the imide bonds provide powerful intermolecular force, it has very high levels of thermal, mechanical and chemical properties.

The porous polyimide film usable for the present invention is a porous polyimide film preferably containing polyimide (as a main component) obtained from tetracarboxylic dianhydride and diamine, more preferably a porous polyimide film composed of tetracarboxylic dianhydride and diamine. The phrase "including as the main component" means that it essentially contains no components other than the polyimide obtained from a tetracarboxylic dianhydride and a diamine, as constituent components of the porous polyimide film, or that it may contain them but they are additional components that do not affect the properties of the polyimide obtained from the tetracarboxylic dianhydride and diamine.

In an embodiment, the porous polyimide film usable for the present invention includes a colored porous polyimide film obtained by forming a polyamic acid solution composition including a polyamic acid solution obtained from a tetracarboxylic acid component and a diamine component, and a coloring precursor, and then heat treating it at 250°C or higher.

A polyamic acid is obtained by polymerization of a tetracarboxylic acid component and a diamine component. A polyamic acid is a polyimide precursor that can be cyclized to a polyimide by thermal imidization or chemical imidization.

The polyamic acid used may be any one that does not have an effect on the invention, even if a portion of the amic acid is imidized. Specifically, the polyamic acid may be partially thermally imidized or chemically imidized.

When the polyamic acid is to be thermally imidized, there may be added to the polyamic acid solution, if necessary, an imidization catalyst, an organic phosphorus-containing compound, or fine particles such as inorganic fine particles or organic fine particles. In addition, when the polyamic acid is to be chemically imidized, there may be added to the polyamic acid solution, if necessary, a chemical imidization agent, a dehydrating agent, or fine particles such as inorganic fine particles or organic fine particles. Even if such components are added to the polyamic acid solution, they are preferably added under conditions that do not cause precipitation of the coloring precursor.

In this specification, a "coloring precursor" is a precursor that generates a colored substance by partial or total carbonization under heat treatment at 250°C or higher.

Coloring precursors usable for the production of the porous polyimide film are preferably uniformly dissolved or dispersed in a polyamic acid solution or polyimide solution and subjected to thermal decomposition by heat treatment at 250°C or higher, preferably 260°C or higher, even more preferably 280°C or higher and more preferably 300°C or higher, and preferably heat treatment in the presence of oxygen such as air, at 250°C, preferably 260°C or higher, even more preferably 280°C or higher and more preferably 300°C or higher, for carbonization to produce a colored substance, more preferably producing a black colored substance, with carbon-based coloring precursors being most preferred.

The coloring precursor, when being heated, first appears as a carbonized compound, but compositionally it contains other elements in addition to carbon, and also includes layered structures, aromatic crosslinked structures and tetrahedron carbon-containing disordered structures.

Carbon-based coloring precursors are not particularly restricted, and for example, they include tar or pitch such as petroleum tar, petroleum pitch, coal tar and coal pitch, coke, polymers obtained from acrylonitrile-containing monomers, ferrocene compounds (ferrocene and ferrocene derivatives), and the like. Of these, polymers obtained from acrylonitrile-containing monomers and/or ferrocene compounds are preferred, with polyacrylonitrile being preferred as a polymer obtained from an acrylonitrile-containing monomer.

Moreover, in another embodiment, examples of the porous polyimide film which may be used for the preset invention also include a porous polyimide film which can be obtained by molding a polyamic acid solution derived from a tetracarboxylic acid component and a diamine component followed by heat treatment without using the coloring precursor.

The porous polyimide film produced without using the coloring precursor may be produced, for example, by casting a polyamic acid solution into a film, the polyamic acid solution being composed of 3 to 60% by mass of polyamic acid having an intrinsic viscosity number of 1.0 to 3.0 and 40 to 97% by mass of an organic polar solvent, immersing or contacting in a coagulating solvent containing water as an essential component, and imidating the porous film of the polyamic acid by heat treatment. In this method, the coagulating solvent containing water as an essential component may be water, or a mixed solution containing 5% by mass or more and less than 100% by mass of water and more than 0% by mass and 95% by mass or less of an organic polar solvent. Further, after the imidation, one surface of the resulting porous polyimide film may be subjected to plasma treatment.

The tetracarboxylic dianhydride which may be used for the production of the porous polyimide film may be any tetracarboxylic dianhydride, selected as appropriate according to the properties desired. Specific examples of tetracarboxylic dianhydrides include biphenyltetracarboxylic dianhydrides such as pyromellitic dianhydride, 3,3',4,4'-biphenyltetracarboxylic dianhydride (s-BPDA) and 2,3,3',4'-biphenyltetracarboxylic dianhydride (a-BPDA), oxydiphthalic dianhydride, diphenylsulfone-3,4,3',4'-tetracarboxylic dianhydride, bis(3,4-dicarboxyphenyl)sulfide dianhydride, 2,2-bis(3,4-dicarboxyphenyl)-1,1,1,3,3,3-hexafluoropropane dianhydride, 2,3,3',4'-benzophenonetetracarboxylic dianhydride, 3,3',4,4'-benzophenonetetracarboxylic dianhydride, bis(3,4-dicarboxyphenyl)methane dianhydride, 2,2-bis(3,4-dicarboxyphenyl)propane dianhydride, p-phenylenebis(trimellitic acid monoester acid anhydride), p-biphenylenebis(trimellitic acid monoester acid anhydride), m-terphenyl-3,4,3',4'-tetracarboxylic dianhydride, p-terphenyl-3,4,3',4'-tetracarboxylic dianhydride, 1,3-bis(3,4-dicarboxyphenoxy)benzene dianhydride, 1,4-bis(3,4-dicarboxyphenoxy)benzene dianhydride, 1,4-bis(3,4-dicarboxyphenoxy)biphenyl dianhydride, 2,2-bis[(3,4-dicarboxyphenoxy)phenyl]propane dianhydride, 2,3,6,7-naphthalenetetracarboxylic dianhydride, 1,4,5,8-naphthalenetetracarboxylic dianhydride, 4,4'-(2,2-hexafluoroisopropylidene)diphthalic dianhydride, and the like. Also preferably used is an aromatic tetracarboxylic acid such as 2,3,3',4'-diphenylsulfonetetracarboxylic acid. These may be used alone or in appropriate combinations of two or more.

Particularly preferred among these are at least one type of aromatic tetracarboxylic dianhydride selected from the group consisting of biphenyltetracarboxylic dianhydride and pyromellitic dianhydride. As a biphenyltetracarboxylic dianhydride there may be suitably used 3,3',4,4'-biphenyltetracarboxylic dianhydride.

As diamine which may be used for the production of the porous polyimide film, any diamine may be used. Specific examples of diamines include the following.

1) Benzenediamines with one benzene nucleus, such as 1,4-diaminobenzene(paraphenylenediamine), 1,3-diaminobenzene, 2,4-diaminotoluene and 2,6-diaminotoluene;

2) diamines with two benzene nuclei, including diaminodiphenyl ethers such as 4,4'-diaminodiphenyl ether and 3,4'-diaminodiphenyl ether, and 4,4'-diaminodiphenylmethane, 3,3'-dimethyl-4,4'-diaminobiphenyl, 2,2'-dimethyl-4,4'-diaminobiphenyl, 2,2'-bis(trifluoromethyl)-4,4'-diaminobiphenyl, 3,3'-dimethyl-4,4'-diaminodiphenylmethane, 3,3'-dicarboxy-4,4'-diaminodiphenylmethane, 3,3',5,5'-tetramethyl-4,4'-diaminodiphenylmethane, bis(4-aminophenyl)sulfide, 4,4'-diaminobenzanilide, 3,3'-dichlorobenzidine, 3,3'-dimethylbenzidine, 2,2'-dimethylbenzidine, 3,3'-dimethoxybenzidine, 2,2'-dimethoxybenzidine, 3,3'-diaminodiphenyl ether, 3,4'-diaminodiphenyl ether, 4,4'-diaminodiphenyl ether, 3,3'-diaminodiphenyl sulfide, 3,4'-diaminodiphenyl sulfide, 4,4'-diaminodiphenyl sulfide, 3,3'-diaminodiphenylsulfone, 3,4'-diaminodiphenylsulfone, 4,4'-diaminodiphenylsulfone, 3,3'-diaminobenzophenone, 3,3'-diamino-4,4'-dichlorobenzophenone, 3,3'-diamino-4,4'-dimethoxybenzophenone, 3,3'-diaminodiphenylmethane, 3,4'-diaminodiphenylmethane, 4,4'-diaminodiphenylmethane, 2,2-bis(3-aminophenyl)propane, 2,2-bis(4-aminophenyl)propane, 2,2-bis(3-aminophenyl)-1,1,1,3,3,3-hexafluoropropane, 2,2-bis(4-aminophenyl)-1,1,1,3,3,3-hexafluoropropane, 3,3'-diaminodiphenyl sulfoxide, 3,4'-diaminodiphenyl sulfoxide and 4,4'-diaminodiphenyl sulfoxide;

3) diamines with three benzene nuclei, including 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene, 1,4-bis(3-aminophenoxy)benzene, 1,4-bis(4-aminophenoxy)benzene, 1,3-bis(3-aminophenoxy)-4-trifluoromethylbenzene, 3,3'-diamino-4-(4-phenyl)phenoxybenzophenone, 3,3'-diamino-4,4'-di(4-phenylphenoxy)benzophenone, 1,3-bis(3-aminophenyl sulfide)benzene, 1,3-bis(4-aminophenyl sulfide)benzene, 1,4-bis(4-aminophenyl sulfide)benzene, 1,3-bis(3-aminophenylsulfone)benzene, 1,3-bis(4-aminophenylsulfone)benzene, 1,4-bis(4-aminophenylsulfone)benzene, 1,3-bis[2-(4-aminophenyl)isopropyl]benzene, 1,4-bis[2-(3-aminophenyl)isopropyl]benzene and 1,4-bis[2-(4-aminophenyl)isopropyl]benzene;

4) diamines with four benzene nuclei, including 3,3'-bis(3-aminophenoxy)biphenyl, 3,3'-bis(4-aminophenoxy)biphenyl, 4,4'-bis(3-aminophenoxy)biphenyl, 4,4'-bis(4-aminophenoxy)biphenyl, bis[3-(3-aminophenoxy)phenyl]ether, bis[3-(4-aminophenoxy)phenyl]ether, bis[4-(3-aminophenoxy)phenyl]ether, bis[4-(4-aminophenoxy)phenyl]ether, bis[3-(3-aminophenoxy)phenyl]ketone, bis[3-(4-aminophenoxy)phenyl]ketone, bis[4-(3-aminophenoxy)phenyl]ketone, bis[4-(4-aminophenoxy)phenyl]ketone, bis[3-(3-aminophenoxy)phenyl] sulfide, bis[3-(4-aminophenoxy)phenyl] sulfide, bis[4-(3-aminophenoxy)phenyl] sulfide, bis[4-(4-aminophenoxy)phenyl] sulfide, bis[3-(3-aminophenoxy)phenyl]sulfone, bis[3-(4-aminophenoxy)phenyl]sulfone, bis[4-(3-aminophenoxy)phenyl]sulfone, bis[4-(4-aminophenoxy)phenyl]sulfone, bis[3-(3-aminophenoxy)phenyl]methane, bis[3-(4-aminophenoxy)phenyl]methane, bis[4-(3-aminophenoxy)phenyl]methane, bis[4-(4-aminophenoxy)phenyl]methane, 2,2-bis[3-(3-aminophenoxy)phenyl]propane, 2,2-bis[3-(4-aminophenoxy)phenyl]propane, 2,2-bis[4-(3-aminophenoxy)phenyl]propane, 2,2-bis[4-(4-aminophenoxy)phenyl]propane, 2,2-bis[3-(3-aminophenoxy)phenyl]-1,1,1,3,3,3-hexafluoropropane, 2,2-bis[3-(4-aminophenoxy)phenyl]-1,1,1,3,3,3-hexafluoropropane, 2,2-bis[4-(3-aminophenoxy)phenyl]-1,1,1,3,3,3-hexafluoropropane and 2,2-bis[4-(4-aminophenoxy)phenyl]-1,1,1,3,3,3-hexafluoropropane.

These may be used alone or in mixtures of two or more. The diamine used may be appropriately selected according to the properties desired.

Preferred among these are aromatic diamine compounds, with 3,3'-diaminodiphenyl ether, 3,4'-diaminodiphenyl ether, 4,4'-diaminodiphenyl ether, paraphenylenediamine, 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene and 1,4-bis(3-aminophenoxy)benzene being preferred for use. Particularly preferred is at least one type of diamine selected from the group consisting of benzenediamines, diaminodiphenyl ethers and bis(aminophenoxy)phenyl.

From the viewpoint of heat resistance and dimensional stability under high temperature, the porous polyimide film which may be used for the invention is preferably formed from a polyimide obtained by combination of a tetracarboxylic dianhydride and a diamine, having a glass transition temperature of 240°C or higher, or without a distinct transition point at 300°C or higher.

From the viewpoint of heat resistance and dimensional stability under high temperature, the porous polyimide film which may be used for the invention is preferably a porous polyimide film comprising one of the following aromatic polyimides.

(i) An aromatic polyimide comprising at least one tetracarboxylic acid unit selected from the group consisting of biphenyltetracarboxylic acid units and pyromellitic acid units, and an aromatic diamine unit,
(ii) an aromatic polyimide comprising a tetracarboxylic acid unit and at least one type of aromatic diamine unit selected from the group consisting of benzenediamine units, diaminodiphenyl ether units and bis(aminophenoxy)phenyl units, and/or,
(iii) an aromatic polyimide comprising at least one type of tetracarboxylic acid unit selected from the group consisting of biphenyltetracarboxylic acid units and pyromellitic acid units, and at least one type of aromatic diamine unit selected from the group consisting of benzenediamine units, diaminodiphenyl ether units and bis(aminophenoxy)phenyl units.

The porous polyimide film used in the present invention is preferably a three-layer structure porous polyimide film having a surface layer A and a surface layer B, the surface layers having a plurality of pores, and a macrovoid layer sandwiched between the surface layers A and B; wherein an average pore diameter of the pores present in the surface layer A is 0.01 $\mu$m to 15 $\mu$m, and the mean pore diameter present on the surface layer B is 20 $\mu$m to 100 $\mu$m; wherein the macrovoid layer has a partition wall bonded to the surface layers A and B, and a plurality of macrovoids surrounded by such a partition wall and the surface layers A and B; wherein the thickness of the macrovoid layer, and the surface layers A and B is 0.01 to 20 $\mu$m, wherein the pores on the surface layers A and B communicate with the macrovoid, the total film thickness is 5 to 500 $\mu$m, and the porosity is 40% or more and less than 95%. In this case, at least one partition wall in the macrovoid layer has one or two or more pores connecting the neighboring macrovoids and having the average pore diameter of 0.01 to 100 $\mu$m, preferably 0.01 to 50 $\mu$m.

For example, porous polyimide films described in WO2010/038873, Japanese Unexamined Patent Publication (Kokai) No. 2011-219585 or Japanese Unexamined Patent Publication (Kokai) No. 2011-219586 may be used for the present invention.

The porous polyethersulfone (PES) film which may be used for the present invention contains polyethersulfone and typically consists substantially of polyethersulfone. Polyethersulfone may be synthesized by the method known to those skilled in the art. For example, it may be produced by a method wherein a dihydric phenol, an alkaline metal compound and a dihalogenodiphenyl compound are subjected to polycondensation reaction in an organic polar solvent, a method wherein an alkaline metal di-salt of a dihydric phenol previously synthesized is subjected to polycondensation reaction dihalogenodiphenyl compound in an organic polar solvent or the like.

Examples of an alkaline metal compound include alkaline metal carbonate, alkaline metal hydroxide, alkaline metal hydride, alkaline metal alkoxide and the like. Particularly, sodium carbonate and potassium carbonate are preferred.

Examples of a dihydric phenol compound include hydroquinone, catechol, resorcin, 4,4'-biphenol, bis (hydroxyphenyl)alkanes (such as 2,2-bis(hydroxyphenyl)propane, and 2,2-bis(hydroxyphenyl)methane), dihydroxydiphenylsulfones, dihydroxydiphenyl ethers, or those mentioned above having at least one hydrogen on the benzene rings thereof substituted with a lower alkyl group such as a methyl group, an ethyl group, or a propyl group, or with a lower alkoxy group such as a methoxy group, or an ethoxy group. As the dihydric phenol compound, two or more of the aforementioned compounds may be mixed and used.

Polyethersulfone may be a commercially available product. Examples of a commercially available product include SUMIKAEXCEL 7600P, SUMIKAEXCEL 5900P (both manufactured by Sumitomo Chemical Company, Limited).

The logarithmic viscosity of the polyethersulfone is preferably 0.5 or more, more preferably 0.55 or more from the viewpoint of favorable formation of a macrovoid of the porous polyethersulfone film; and it is preferably 1.0 or less, more preferably 0.9 or less, further preferably 0.8 or less, particularly preferably 0.75 or less from the viewpoint of the easy production of a porous polyethersulfone film.

Further, from the viewpoints of heat resistance and dimensional stability under high temperature, it is preferred that the porous polyethersulfone (PES) film or polyethersulfone as a raw material thereof has a glass transition temperature of 200°C or higher, or that a distinct glass transition temperature is not observed.

The method for producing the porous polyethersulfone (PES) film which may be used for the present invention is not particularly limited. For example, the film may be produced by a method including the following steps:

a step in which polyethersulfone solution containing 0.3 to 60% by mass of polyethersulfone having logarithmic viscosity of 0.5 to 1.0 and 40 to 99.7% by mass of an organic polar solvent is casted into a film, immersed in or contacted with a coagulating solvent containing a poor solvent or non-solvent of polyethersulfone to produce a coagulated film having pores; and
a step in which the coagulated film having pores obtained in the above-mentioned step is heat-treated for coarsening of the aforementioned pores to obtain a porous polyethersulfone (PES) film;
wherein the heat treatment includes the temperature of the coagulated film having the pores is raised higher than the glass transition temperature of the polyethersulfone, or up to 240°C or higher.

The porous polyethersulfone (PES) film which can be used in the present invention is preferably a porous polyethersulfone (PES) film having a surface layer A, a surface layer B, and a macrovoid layer sandwiched between the surface layers A and B,
wherein the macrovoid layer has a partition wall bonded to the surface layers A and B, and a plurality of macrovoids surrounded by such a partition wall and the surface layers A and B, the macrovoids having the average pore diameter in the planar direction of the film of 10 to 500 $\mu$m;
wherein the thickness of the macrovoid layer is 0.1 to 50 $\mu$m,
each of the surface layers A and B has a thickness of 0.1 to 50 $\mu$m,
wherein one of the surface layers A and B has a plurality of pores having the average pore diameter of more than 5 $\mu$m and 200 $\mu$m or less, while the other has a plurality of pores having the average pore diameter of 0.01 $\mu$m or more and less than 200 $\mu$m,
wherein one of the surface layers A and B has a surface aperture ratio of 15% or more while other has a surface aperture ratio of 10% or more,
wherein the pores of the surface layers A and B communicate with the macrovoids,
wherein the porous polyethersulfone (PES) film has total film thickness of 5 to 500 $\mu$m and a porosity of 50 to 95%.

5. Cell culture and culture volume

FIG. 1 represents a model diagram of cell culturing using a porous polymer film. FIG. 1 serves merely for illustration and the elements are not drawn to their actual dimensions. In the cell culture method of the invention, application of cells and culturing are carried out on a porous polymer film, thereby allowing culturing of large volumes of cells to be accomplished since large numbers of cells grow on the multisided connected pore sections on the inside, and the surfaces on the porous polymer film. Moreover, in the cell culture method of the invention, it is possible to culture large volumes of cells while drastically reducing the amount of medium used for cell culturing compared to the prior art. For example, large volumes of cells can be cultured even when all or a portion of the porous polymer film is not in contact with the liquid phase of the cell culture medium. In addition, the total volume of the cell culture medium in the cell culture vessel, with respect to the total porous polymer film volume including the cell survival zone, can be significantly reduced.
Throughout the present specification, the volume of the porous polymer film without cells, that occupies the space including the volume between the interior gaps, will be referred to as the "apparent porous polymer film volume" (see, FIG. 1). In the state where the cells are applied to the porous polymer film and the cells have been supported on the surface and the interior of the porous polymer film, the total volume of the porous polymer film, the cells and the medium that has wetted the porous polymer film interior, which is occupying the space therein, will be referred to as the "porous polymer film volume including the cell survival zone" (see, FIG. 1). When the porous polymer film has a film thickness of 25 $\mu$m, the porous polymer film volume including the cell survival zone is a value of at maximum about 50% larger than the apparent porous polymer film volume. In the method of the invention, a plurality of porous polymer films may be housed in a single cell culture vessel for culturing, in which case the total sum of the porous polymer film volume including the cell survival zone for each of the plurality of porous polymer films supporting the cells may be referred to as simply the "total sum of the porous polymer film volume including the cell survival zone".
Using the method of the invention, cells can be satisfactorily cultured for a long period of time even under conditions in which the total volume of the cell culture medium in the cell culture vessel is 10,000 times or less of the total sum of the porous polymer film volume including the cell survival zone. Moreover, cells can be satisfactorily cultured for a long period of time even under conditions in which the total volume of the cell culture medium in the cell culture vessel is 1,000 times or less of the total sum of the porous polymer film volume including the cell survival zone. In addition, cells can be satisfactorily cultured for a long period of time even under conditions in which the total volume of the cell culture medium in the cell culture vessel is 100 times or less of the total sum of the porous polymer film volume including the cell survival zone. In addition, cells can be satisfactorily cultured for a long period of time even under conditions in which the total volume of the cell culture medium in the cell culture vessel is 10 times or less of the total sum of the porous polymer film volume including the cell survival zone.

In other words, according to the invention, the space (vessel) used for cell culturing can be reduced to an absolute minimum, compared to a conventional cell culture device for performing two-dimensional culture. Furthermore, when it is desired to increase the number of cells cultured, the cell culturing volume can be flexibly increased by a convenient procedure including increasing the number of layered porous polymer films. In a cell culture device comprising a porous polymer film to be used for the invention, the space (vessel) in which cells are cultured and the space (vessel) in which the cell culture medium is stored can be separate, and the necessary amount of cell culture medium can be prepared according to the number of cells to be cultured. The space (vessel) in which the cell culture medium is stored can be increased or decreased according to the purpose, or it may be a replaceable vessel, with no particular restrictions.

In the cell culture method of the invention, culturing in which the number of cells in the cell culture vessel after culturing using the porous polymer film reaches $1.0 \times 10^5$ or more, $1.0 \times 10^6$ or more, $2.0 \times 10^6$ or more, $5.0 \times 10^6$ or more, $1.0 \times 10^7$ or more, $2.0 \times 10^7$ or more, $5.0 \times 10^7$ or more, $1.0 \times 10^8$ or more, $2.0 \times 10^8$ or more, $5.0 \times 10^8$ or more, $1.0 \times 10^9$ or more, $2.0 \times 10^9$ or more, or $5.0 \times 10^9$ or more per milliliter of medium, assuming that all of the cells are evenly dispersed in the cell culture medium in the cell culture vessel, is mentioned.

It should be noted that as a method for measuring cell count during or after culture, various known methods may be used. For example, as the method for counting the number of cells in the cell culture vessel after culturing using the porous polymer film, assuming that the cells are evenly dispersed in the cell culture medium in the cell culture vessel, any publicly known method may be used. For example, a cell count method using CCK8 may be suitably used. Specifically, a Cell Counting Kit 8 (a solution reagent, commercially available from Dojindo Laboratories)(hereunder referred to as "CCK8") may be used to count the number of cells in ordinary culturing without using a porous polymer film, and the correlation coefficient between the absorbance and the actual cell count is determined. Subsequently, the cells are applied, the cultured porous polymer film may be transferred to CCK8-containing medium and stored in an incubator for 1 to 3 hours, and then the supernatant is extracted and its absorbance is measured at a wavelength of 480 nm, and the cell count is determined from the previously calculated correlation coefficient.

In addition, from another point of view, for example, "mass culturing of cells" may refer to culturing in which the number of cells in the cell culture vessel after culturing using the porous polyimide film reaches $1.0 \times 10^5$ or more, $2.0 \times 10^5$ or more, $1.0 \times 10^6$ or more, $2.0 \times 10^6$ or more, $5.0 \times 10^6$ or more, $1.0 \times 10^7$ or more, $2.0 \times 10^7$ or more or $5.0 \times 10^7$ or more, $1.0 \times 10^8$ or more, $2.0 \times 10^8$ or more, or $5.0 \times 10^8$ or more, per square centimeter of porous polymer film. The number of cells contained per square centimeter of porous polymer film may be appropriately measured using a publicly known method, such as with a cell counter.


6. Culture system and culture conditions of neural stem cells

In the methods of the present invention, the cell culture medium for the neural stem cells is not particularly limited provided the cells can be cultured. However, a culture medium in which $1 \times$ N2 supplement, $0.5 \times$ B27 supplement, 25 ng/ml bFGF, and 25 ng/ml EGF are added to DMEM/F-12HAM can be used.

Culturing of cells using a porous polymer film can be carried out together with other suspension culture carriers such as microcarriers or a cellulose sponge.

In the methods of the present invention, the shape and scale of the system used for culturing is not particularly limited and anything from petri dishes, flasks, plastic bags, and test tubes to large scale tanks for culturing cells can be appropriately used. These include, for example, cell culture dishes manufactured by BD Falcon and Nunc Cell Factory manufactured by Thermo Scientific. Note that, for example, spinner flasks manufactured by Corning or rotating cultures can be used as the apparatus for suspension culture. Further, a hollow fiber culture system such as Fiber Cell™ System manufactured by Veritas may also be used to provide an environment that can achieve a similar function.

The method of the present invention can be carried out using a cell culture module comprising a porous polymer film and a casing housing the porous polymer film having two or more culture medium flow ports wherein

    (i) two or more independent porous polymer films are aggregated,
    (ii) the porous polymer film is folded up,
    (iii) the porous polymer film is wound into a roll shape, and/or
    (iv) the porous polymer film is tied in a rope,

and stored in the casing. The "cell culture module" described herein refers to a cell culture substrate that can be used in cell culture vessels, cell culture apparatuses and cell culture systems, particularly in cell culture vessels, cell culture apparatuses and cell culture systems for suspension culture. By bringing the neural stem cell-containing suspension in contact with the cell culture module the neural stem cells adhere to the porous polymer film. The neural stem cells are made to proliferate and thereafter, those released as spheroids are harvested or reinoculated. Therefore, the harvesting of a large amount of neural stem cells is feasible.

By housing the porous polymer film in a casing, the deformation of the shape of the film-like porous polymer film can constantly be prevented in the casing. The application of stress on the neural stem cells grown in the porous polymer film can thereby be prevented, apoptosis and the like can be averted, and a large amount of neural stem cells can be stably cultured.

The casing provided with the cell culture module has two or more culture medium flow ports that allow the cell culture medium to be supplied into the casing and discharged out of the casing. It is preferable for the diameter of the culture medium flow ports to be larger than the dimeter of the cells to allow the inflow of cells into the interior of the casing. Further, it is preferable for the diameter of the culture medium flow ports to be smaller than a diameter that would allow the outflow of the porous polymer film through the culture medium flow ports. A diameter smaller than the diameter through which the porous polymer film can flow out can be appropriately selected according to the size and shape of the porous polymer film housed in the casing. For example, if the porous polymer film is string-shaped, other than having an appropriate diameter smaller than the width of the short side of the porous polymer film through which the porous polymer film will not flow out, there are no particular limitations. It is preferable for the number of culture medium flow ports to be as large as possible in order to facilitate the supply and/or discharge of the cell medium into/out of the casing. Preferably 5 or more, preferably 10 or more, preferably 20 or more, preferably 50 or more, preferably 100 or more are provided. The medium flow ports may include a mesh-shaped structure over a part or the whole of the casing. Further, the casing itself may be mesh shaped. Herein, the "mesh-shaped structure" comprises, for example, a vertical, horizontal and/or diagonal lattice-like structure, each opening forming a medium flow port through which a fluid can flow through but is not limited thereto.

The casing provided with the aforementioned cell culture module is preferably formed of a non-flexible material preferably having sufficient strength not to be deformed by the movement of culture medium under normal culture conditions, for example, stirring or shaking culture conditions. Further, the casing is preferably formed of a material that does not affect the growth of cells during cell culture. Such materials include but are not limited to, for example, polymers such as polyethylene, polypropylene, nylon, polyester, polystyrene, polycarbonate, polymethyl methacrylate, polyethylene terephthalate, and metals such as stainless steel and titanium. The casing requires a certain degree of strength to continuously prevent the shape of the porous polymer film in the casing from being deformed. Herein, the "casing does not deform" has the meaning of the casing not deforming under loads received in a normal culturing environment and does not mean that the casing will never deform.

In the cell culture module:

(i) two or more independent porous polymer films are aggregated;
(ii) the porous polymer film is folded up;
(iii) the porous polymer film is wound into a roll shape; and/or
(iv) the porous polymer film is tied in a rope

and stored in the casing.

Herein, "two or more independent porous polymer films are aggregated and stored in the casing" indicates a state wherein two or more mutually independent porous polymer films are aggregated and stored in a fixed space enclosed by the casing. In the aforementioned cell culture module two or more independent porous polymer films may be secured such that the porous polymer film does not move within the casing by securing at least one part of the porous polymer film to at least one part within the casing by an arbitrary method. Further, the two or more independent porous polymer films may be small pieces. The shape of the small pieces may be any shape such as a circle, ellipse, quadrilateral, triangle, polygonal or string-like but is preferably a square. In the cell culture module, the size of the small piece may be any size, and in the case of a square, the length may be any length but is preferably 80 mm or less, preferably 30 mm or less, and more preferably 10 mm or less. The application of stress on the cells grown in the porous polymer film can thereby be prevented, apoptosis and the like can be averted, and a large amount of cells can be stably cultured. Note that in the case of string-like porous polymer films, the porous polymer film may be housed in the casing so as to be (ii) folded up, (iii) wound into a roll shape and/or (iv) tied in a rope shape, as will be discussed later.

Herein, the "folded-up porous polymer film" refers to a porous polymer film that has been folded up and is in the casing such that the porous polymer film does not move within the casing due to frictional forces on each surface of the porous polymer film and/or the internal surfaces of the casing. Herein, "folded-up" may refer to a state in which there are fold lines in the porous polymer film or to a state in which there are no fold lines therein.

Herein, the "porous polymer film wound into a roll shape" refers to a porous polymer film that has been wound into a roll shape and is in the casing such that the porous polymer film does not move within the casing due to frictional forces on each surface of the porous polymer film and/or the internal surfaces of the casing. Furthermore, herein, the "porous polymer film woven into a rope shape" refers to, for example, a plurality of porous polymer film strips woven into a rope shape by an arbitrary method such that the porous polymer films are in a state in which the frictional forces therebetween prevent the movement thereof. A combination wherein (i) two or more independent porous polymer films are aggregated, (ii) the porous polymer film is folded up, (iii) the porous polymer film is wound into a roll shape and/or (iv) the porous polymer film is tied in a rope shape may be carried out and then stored in the casing.

Herein, the "porous polymer film does not move within the casing" refers to a state wherein the porous polymer film is housed in the casing such that the porous polymer film continues to remain in a non-deformed state when culturing with the cell culture module in the cell culture medium. In other words, the porous polymer film itself is in an inhibited state so that the occurrence of ripple-like movement caused by the fluid is continuously inhibited. Since the state in which the porous polymer film does not move within the casing is maintained, stress is prevented from being applied to cells grown within the porous polymer film, and cells can be stably cultured without cells dying due to apoptosis and the like.

The aforementioned cell culture module may be a commercially available culture apparatus, system, or the like which can culture cells in suspension. For example, the module can be used in a culture apparatus in which a culture vessel comprises a flexible bag and can be used in a state where cells are suspended in the culture vessel. Furthermore, the cell culture module can culture cells by being used in an agitating culture vessel such as a spinner flask. In addition, the module may be used in culture vessels that are open vessels or closed vessels. For example, anything from petri dishes, flasks, plastic bags, and test tubes to large scale tanks for culturing cells can be appropriately used. These include, for example, cell culture dishes manufactured by BD Falcon and Nunc Cell Factory manufactured by Thermo Scientific.

7. Harvesting of cultured cell

In one embodiment, the neural stem cells cultured by the method of the present invention are harvested. There are no particular limitations to the harvesting method. However, neural stem cells that have formed spheroids and have been released from the porous polymer film into the culture medium may be harvested. Alternatively, spheroids of neural stem cells attached to the porous polymer film may be harvested by releasing the spheroids from the porous polymer film by, for example, pipetting. Furthermore, the neural stem cells can be efficiently harvested or reinoculated using a method in which the porous polymer film is treated with an enzyme such as Acutase™ (Nacalai Tesque).

8. Method for inducing differentiation of the present invention

In the method for inducing differentiation of the present invention, neural stem cells that were made to proliferate on the porous polymer film are induced to differentiate by culturing under differentiation-inducing conditions. There are no particular limitations as to the specific steps involved in the induction of differentiation of the neural stem cells. Including the steps described herein, any method suitable for exposing neural stem cells grown on a membrane-like carrier to a differentiation inducing environment such as a differentiation inducing culture medium can be used.

Although in no way limited, differentiation of neural stem cells into neurons and glial cells can be carried out by methods that are well known to a person skilled in the art, and can be carried out using, for example, PDGF, forskolin, BMP2, and the like although not limited thereto.

The present invention will be described in more detail based on the Examples. Note that the present invention is not limited by the Examples. A person skilled in the art could easily add modifications or changes to the present invention based on the disclosures herein and these would be included within the technical scope of the present invention.

EXAMPLES

The porous polyimide film used in the following examples was prepared by forming a polyamic acid solution composition comprising a polyacrylamide which is a coloring precursor and a polyamic acid solution obtained from 3,3', 4,4'-biphenyltetracarboxylic acid dianhydride (s-BPDA) which is a tetracarboxylic acid component and 4, 4'-diaminodiphenyl ether (ODA) which is a diamine component, and thereafter performing heat treatment at 250 °C. The obtained porous polyimide film was a three-layered structure comprising a surface layer A and a surface layer B having a plurality of pores and a macrovoid layer interposed between the surface layer A and the surface layer B wherein the average pore diameter of the pores present in the surface A was 6 $\mu$m, the average pore diameter of pores present in the surface layer B was 46 $\mu$m, the membrane thickness was 25 $\mu$m, and the porosity was 73%.

EXAMPLE 1

Culturing of neural stem cells on the porous polyimide film

Neural stem cells were extracted from the hippocampus including the dentate gyrus of a wild type mouse (C57BL/6J acquired from CLEA Japan, Inc.). The acquired neural stem cells were cultured for eight days in a vessel (a tissue culture flask with a vented cap (surface treated for adhesive cells) manufactured by AGC Inc.) in which was contained 10 ml of a cell culture medium (D-MEM/Ham's F-12, Wako pure chemical corporation). Neural stem cell spheroids were harvested from the culture medium, the spheroids were degraded by treatment with an enzyme (Acutase™ (Nacalai Tesque), and a cell culture medium suspension containing neural stem cells was prepared. The aforementioned neural stem cell suspension was added to a vessel (a tissue culture dish (surface treated for adhesive cells) manufactured by AGC Inc.) containing a sterilized 1.8 cm$^2$ square-shaped porous polyimide film and culture medium (D-MEM/Ham's F-12, Wako chemical corporation) and culturing was started. The porous polyimide film was transferred into a vessel containing new cell culture medium 10 days after culturing was started. Three days thereafter, the porous polyimide film was transferred to a vessel containing new cell culture medium. FIG. 2 shows an optical micrograph of neural stem cells ten days after culturing was started. Further, neural stem cell spheroids released in the cell culture medium were harvested 10 days after and 70 days after the start of culturing and were immunostained. Immunostaining was carried out on the cells by fixing with 4% PFA, treating with 0.1 % triton X-100, performing blocking thereon, and thereafter reacting with primary antibodies overnight at 4 °C and then with secondary antibodies for one hour at room temperature. FIG. 3 and FIG. 4 show photomicrographs thereof. Even after 70 days the neural stem cells were maintained in an undifferentiated state (FIG. 4). Further, 30 days after the start of culturing, the indicator of DNA synthesis EdU was incorporated for a short time into the spheroids growing on porous polyimide film and the mitotic S phase cell nucleus was fluorescently labelled (FIG. 5 and 6). It was shown that cells were proliferating inside and outside the porous polyimide film.

EXAMPLE 2

Inducing differentiation of neural stem cells cultured on the porous polyimide film

In Example 1, a differentiation inducing agent (PDGF, forskolin, or BMP2) was added to the neural stem cell spheroids harvested after 10 days and after 70 days from the start of culturing and immunostaining was carried out after culturing for five days. Photomicrographs thereof are shown in FIGS. 7 to 13. It is shown that even 70 days after the start of culturing the ability of the neural stem cells to differentiate was maintained (FIGS. 10 to 13).

EXAMPLE 3

Long term culturing of neural stem cells on a porous polyimide film and induction of differentiation of neural stem cells after long term culturing
According to Example 1, neural stem cells were cultured on a 1.8 cm$^2$ square-shaped porous polyimide film. 30 days after the start of culturing, a separately prepared 1.8 cm$^2$ square-shaped porous polyimide film was placed on the porous polyimide film on which the neural stem cells were cultured such that the A surface of both porous polyimide films overlapped and culturing was continued. By adding a new porous polyimide film the space available for culturing was increased. Thereafter, after every seven days a separately prepared 1.8 cm$^2$ square-shaped porous polyimide film was placed on one of the porous polyimide films on which the neural stem cells were cultured such that the A surface of both porous polyimide films overlapped, and by continuing culturing while appropriately increasing the space available for culturing, culturing of the neural stem cells could ultimately be carried out for 225 days.
A differentiation inducing agent (PDGF or BMP2) was added to neural stem cell spheroids harvested 225 days after the start of culturing, and after culturing for five days immunostaining was carried out. FIGS. 14 to 16 show photomicrographs thereof. It is shown that even 225 days after the start of culturing the ability of the neural stem cells to differentiate was maintained.

INDUSTRIAL APPLICABILITY

The method of the present invention inhibits the differentiation of neural stem cells and can be used to supply the cells in a large amount.

**Claims**

1.   A method for inhibiting differentiation of neural stem cells comprising the steps:

        (1) applying neural stem cells to a porous polymer film; and
        (2) culturing and proliferating the neural stem cells,

   wherein the porous polymer films are a three-layer structure porous polymer film having a surface layer A and a surface layer B, the surface layers having a plurality of pores, and a macrovoid layer sandwiched between the surface layers A and B, wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by such a partition wall and the surface layers A and B; and wherein the pores in the surface layers A and B communicate with the macrovoid.

2. The method according to claim 1, wherein the neural stem cells are wild-type cells.

3. The method according to claim 1 or 2, wherein the step (2) is implemented for at least 70 days.

4. The method according to any one of claims 1 to 3, wherein the step (2) is implemented until the neural stem cells have proliferated to at least $1.0 \times 10^5$ per square centimeter of the porous polymer film.

5. The method according to any one of claims 1 to 4, using two or more porous polymer films layered either above and below or left and right in the cell culture medium.

6. The method according to any one of claims 1 to 5, wherein the porous polymer film is:

> i) folded;
> ii) wound into a roll;
> iii) connected as sheets or fragments by a filamentous structure, or
> iv) bound into a rope,

and suspended or anchored in a cell culture medium in a cell culturing vessel.

7. The method according to any one of claims 1 to 6, wherein in the step (2), the total volume of the cell culture medium contained in the cell culturing vessel is 10,000 times or less than the total volume of the porous polyimide film including a cell survival zone.

8. The method according to any one of claims 1 to 7, wherein the average pore diameter of the surface layer A is 5 $\mu$m to 50 $\mu$m.

9. The method according to any one of claims 1 to 8, wherein the average pore diameter of the surface layer B is 20 to 100 $\mu$m.

10. The method according to any one of claims 1 to 9, wherein the thickness of the porous polymer film is 5 to 500 $\mu$m.

11. The method according to any one of claims 1 to 10, wherein the porous polymer film is a porous polyimide film.

12. The method according to claim 11, wherein the porous polyimide film is a porous polyimide film comprising a polyimide derived from tetracarboxylic acid dianhydride and diamine.

13. The method according to claim 11 or 12, wherein the porous polyimide film is a colored porous polyimide film that is obtained by molding a polyamic acid solution composition comprising a polyamic acid solution derived from tetracarboxylic dianhydride and diamine, and a coloring precursor, and subsequently heat-treating the resultant composition at 250°C or higher.

14. The method according to any one of claims 1 to 10, wherein the porous polymer film is a porous polyethersulfone film.

15. A method for preparing neural stem cells comprising the steps:

> (1) applying neural stem cells to a porous polymer film; and
> (2) culturing and proliferating the neural stem cells,

wherein the porous polymer films are a three-layer structure porous polymer film having a surface layer A and a surface layer B, the surface layers having a plurality of pores, and a macrovoid layer sandwiched between the surface layers A and B, wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by such a partition wall and the surface layers A and B; wherein the pores in the surface layers A and B communicate with the macrovoid; and wherein the differentiation of neural stem cells is inhibited in the step (2).

16. The method according to claim 15, further comprising the step of harvesting neural stem cells obtained in the step (2).

17. A method for inducing differentiation of neural stem cells comprising the steps:

(1) applying neural stem cells to a porous polymer film;
(2) culturing and proliferating the neural stem cells; and
(3) culturing the neural stem cells obtained in the step (2) under differentiation-inducing conditions

wherein the porous polymer films are a three-layer structure porous polymer film having a surface layer A and a surface layer B, the surface layers having a plurality of pores, and a macrovoid layer sandwiched between the surface layers A and B, wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by such a partition wall and the surface layers A and B; wherein the pores in the surface layers A and B communicate with the macrovoid; and wherein the differentiation of neural stem cells is inhibited in the step (2).

# FIG. 1

Mesh surface

Large hole surface

Apparent member volume

Member volume comprising cell viable region

# FIG. 2

Porous film

200 μm

FIG. 3

FIG. 4

FIG. 5

# FIG. 6

# FIG. 7

| DAPI | Neuronal mark | GFAP | Merged |

FIG. 8

FIG. 9

FIG. 10

FIG. 11

FIG. 12

FIG. 13

FIG. 14

# FIG. 15

# FIG. 16

## INTERNATIONAL SEARCH REPORT

| International application No. |
|---|
| PCT/JP2018/011492 |

A. CLASSIFICATION OF SUBJECT MATTER
Int.Cl. C12N5/0797(2010.01)i

According to International Patent Classification (IPC) or to both national classification and IPC

B. FIELDS SEARCHED

Minimum documentation searched (classification system followed by classification symbols)
Int.Cl. C12N5/0797

Documentation searched other than minimum documentation to the extent that such documents are included in the fields searched
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X | WO 2015/012415 A1 (UBE INDUSTRIES, LTD.) 29 January 2015, claims, paragraphs [0054], [0068], [0072]-[0074], [0093]-[0095], [0098], examples & US 2016/0168560 A1, claims, paragraphs [0095], [0110], [0113]-[0115], [0137]-[0139], [0142], examples & EP 3026108 A1 | 1-17 |
| A | JP 2014-60991 A (ASAHI KASEI CORPORATION) 10 April 2014 (Family: none) | 1-17 |
| A | JP 2008-54621 A (HOKKAIDO UNIVERSITY) 13 March 2008 (Family: none) | 1-17 |
| P, X | WO 2018/021357 A1 (UBE INDUSTRIES, LTD.) 01 February 2018, claims (Family: none) | 1-17 |

☐ Further documents are listed in the continuation of Box C.  ☐ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 05 June 2018 (05.06.2018) | 12 June 2018 (12.06.2018) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008054621 A
- WO 2010038873 A
- JP 2011219585 A
- JP 2011219586 A
- WO 2015012415 A

**Non-patent literature cited in the description**

- **SEABERG, M. et al.** *The Journal of Neuroscience,* 01 March 2002, vol. 22 (5), 1784-1793
- **YOSHIOKA, K. et al.** *FEBS Open Bio,* 2015, vol. 5, 437-444